# DEMANDE DE BREVET EUROPEEN

(11) **EP 3 075 387 A1**
(43) Date de publication de la demande: **05.10.2016**
(21) Numéro de dépôt: 16162601.5
(22) Date de dépôt: 15.12.2006
(51) Int. Cl.: A61K 38/36, A61P 7/04, A61L 24/10

(54) **COLLE BIOLOGIQUE EXEMPTE DE THROMBINE ET SON UTILISATION COMME MEDICAMENT**

(30) Priorité: 16.12.2005 FR 0512817
(62) Demande divisionnaire de: 06841950.6
(71) Demandeur: Laboratoire Français du Fractionnement et des Biotechnologies, 91940 Les Ulis (FR)
(72) Inventeur: CHTOUROU, Abdessatar, 78990 ELANCOURT (FR)
(74) Mandataire: Lavoix

(57) **Abrégé**

L'invention concerne une colle biologique à base de fibrinogène, à usage thérapeutique, comprenant du facteur VIIa et une source d'ions calcium, et est exempte de thrombine. L'invention concerne également l'utilisation de la colle biologique comme médicament, notamment comme pansement pour les tissus biologiques, les plaies ou les biomatériaux.

## Description

La présente invention se rapporte à une colle biologique à base de fibrinogène, de facteur VII activé et une source d'ions calcium.

On entend par « colle biologique » un composant capable de réunir des éléments tissulaires (peau, os, organes divers) et en même temps d'assurer une hémostase des tissus lésés, pouvant ainsi renforcer ou compléter une réunion de ces tissus par une ou des sutures.

La coagulation sanguine est réalisée selon des étapes en cascade impliquant différentes proenzymes et procofacteurs présents dans le sang qui sont converties, par l'intermédiaire d'enzymes protéolytiques, en leur forme activée. Cette succession d'étapes ou cascade de la coagulation est effectuée selon deux systèmes de la coagulation, appelés voie extrinsèque de la coagulation et voie intrinsèque de la coagulation, conduisant à la transformation de la prothrombine en thrombine.

La voie extrinsèque implique l'intervention du Facteur VII présent dans le sang. Toutefois, celui-ci requiert une activation (facteur VIIa) pour initier cette cascade de coagulation. Le facteur VIIa présente une faible activité enzymatique jusqu'à ce qu'il se complexe aux facteurs tissulaires de nature phospholipidiques libérés après lésion tissulaire. Le facteur VIIa ainsi complexé transforme le facteur X (FX) en facteur Xa (FXa) en présence d'ions calcium. Le facteur Xa à son tour transforme la prothrombine en thrombine, laquelle active le facteur V (Facteur Va). La thrombine active également le facteur XIII (facteur XIIIa). La thrombine, en présence de calcium, de facteurs tissulaires, de facteur Va, agit sur le fibrinogène en le transformant en fibrine. La présence de facteur XIIIa permet la formation d'un caillot de fibrine à réseau maillé solide et adhérent qui est progressivement et lentement résorbé avec l'installation du tissu cicatriciel de consolidation, à laquelle le réseau sert de trame. Cette fibrine réticulée est insoluble et n'est pas attaquable par les enzymes fibrinolytiques, au moins durant le temps de mise en place du tissu cicatriciel.

La voie intrinsèque de la coagulation implique également le facteur VIIa (FVIIa). Cette voie comprend une cascade de réactions aboutissant à l'activation de la thrombine par l'intermédiaire du facteur XII (FXII). Celui-ci active le facteur XI (Facteur XIa - FXIa) qui active le facteur IX (facteur IXa - FIXa) en présence de facteurs tissulaires phospholipidiques (FT). Le facteur IXa participe à l'activation du facteur X en facteur Xa en présence de facteur VIIIa, des facteurs tissulaires et d'ions calcium. Ceci conduit ensuite à la transformation de la prothrombine en thrombine. Il convient de noter que la présence de facteur Xa ou de thrombine permet l'activation du facteur VII (facteur VIIa).

Il apparait donc que le facteur VIIa joue un rôle prépondérant dans les mécanismes de la coagulation intrinsèque, aboutissant à la formation de caillot sanguin. Il est utilisé dans le traitement des hémophiles A ayant un inhibiteur circulant, c'est-à-dire un anticorps spécifique qui limite ou empêche l'activation du facteur VIII (FVIII). Le facteur VIIa présente l'intérêt de pouvoir agir localement en présence de facteurs tissulaires libérés après lésion de tissus engendrant des hémorragies, même en l'absence de Facteur VIII ou IX.

Cependant, dans le cas de lésions tissulaires hémorragiques engendrées par les plaies, blessures ou interventions chirurgicales externes, la réparation tissulaire par suture est une nécessité pour notamment faire cesser l'hémorragie par formation du caillot de fibrine, selon les mécanismes explicités précédemment. Il peut s'avérer nécessaire de favoriser, notamment dans le cas de chirurgies lourdes, l'hémostase de tissus lésés.

On peut citer, à titre d'exemple, la demande de brevet WO93/06855 qui décrit une composition hémostatique, exempte de thrombine et de facteurs de la coagulation, comprenant du facteur VIIa incorporé dans un véhicule compatible sur le plan biologique, l'ensemble étant applique sur une blessure hémorragique pour en favoriser l'hémostase et la formation de caillot de fibrine.

Cependant, hormis l'hémostase de tissus lésés, il convient également d'accélérer, renforcer, voire compléter le processus naturel de cicatrisation tissulaire, suturé ou non, liée à la réunion des tissus pour éviter leur déshience et accélérer le processus. Ceci est notamment possible par l'application locale de colle biologique.

Les colles biologiques sont constituées d'un mélange de facteurs circulants de la coagulation sanguine, notamment de protéines plasmatiques fibrinogène et facteur XIII. Elles requièrent en outre un apport de thrombine exogène, enzyme nécessaire pour transformer le fibrinogène en fibrine insoluble, réticulable par le facteur XIII. Des composants additionnels sont nécessaires pour effecteur la coagulation du fibrinogène, notamment l'ion calcium, sous forme de CaCl₂, et éventuellement de l'aprotinine, ajoutée pour ses propriétés anti-fibrinolytiques.

De telles colles biologiques ont été en particulier décrites dans différentes publications et brevets, tels que EP 0 305 243, FR 2 448 900 et FR 2 448 901, ainsi que leurs applications dans diverses situations cliniques (« Fibrinkleber », Actes du Congrès de Heidelberg, 1976, Ed. Schattauer).

Les colles biologiques peuvent également servir à fixer des biomatériaux de nature diverse (collagène, alginates et acide polylactique) à des tissus biologiques pour renforcer leurs propriétés mécaniques en attendant une consolidation par la repousse naturelle des cellules de l'organisme, par exemple peau artificielle.

Les colles biologiques disponibles dans le commerce se présentent en réalité sous forme de kits comprenant au moins les quatre composants cités ci-dessus sous forme sèche dans lequel les protéines coagulables par la thrombine, le fibrinogène et le facteur XIII, doivent être isolés de la thrombine, car leur association engendre une prise en fibrine en un temps très bref, de l'ordre de quelques secondes après reconstitution dans un liquide et mélange. C'est la raison pour laquelle les kits de colle biologique sont au moins bi-composants, c'est-à-dire comprenant, d'une part, un lot à base de fibrinogène et de facteur XIII et, d'autre part, un lot à base de thrombine. La colle biologique remplit ses fonctions indiquées ci-dessus par reconstitution et mélange des deux lots, par exemple avec des seringues et aiguilles, puis application sur le tissu à suturer.

Cependant, de telles reconstitutions et mélanges sont relativement complexes et source d'erreurs possibles, notamment en cas d'urgence, étant donné que dès la mise en contact du fibrinogène et de la thrombine sous forme liquide, en présence des autres composants, il se forme presque instantanément de la fibrine. Le mélange fibrinogène-thrombine ne peut donc se faire que juste avant l'application sur la plaie ou zone opératoire. De plus, lorsqu'un dispositif unique de distribution de colle biologique est utilisé, il existe un risque réel d'une prise en fibrine dans le dispositif même, ce qui peut conduire à un colmatage du système de distribution et d'application.

Le brevet EP 0 850 650 B1 décrit notamment une colle bi-composant préactivée contenant du fibrinogène et au moins un facteur de la coagulation activé, dont l'activation ne dépend pas des ions calcium, choisi parmi le Facteur XIIa, le Facteur XIa, le Facteur VIIa et la kallicréine et elle coagule par simple ajout d'ions calcium par formation de fibrine. Cette colle est obtenue par fractionnement ou concentration du plasma sanguin activé, provenant d'un seul donneur, de façon à concentrer le fibrinogène tout en conservant les facteurs de la coagulation activés nécessaires pour permettre la coagulation sous l'action des ions calcium. Une telle colle présente les inconvénients des colles bi-composants cites ci-dessus et de former le caillot de fibrine quasi instantanément au mélange.

La demande de brevet WO 02/055102 A1 décrit une composition comprenant du FVIIa et du fibrinogène utile pour limiter ou arrêter le saignement provoqué par des hémorragies ou blessures internes ou externes. Ces compositions sont destinées à être injectées par voie intraveineuse au patient et elles agissent au niveau de l'hémorragie ou de la blessure où sont présents tous les facteurs et les ions calcium. L'inconvénient lié à l'utilisation d'une telle composition réside dans le fait que celle-ci peut interagir avec les FT qui se seraient répandus hors de la blessure ou de l'hémorragie, par circulation sanguine par exemple, et engendrer, avec les ions calcium naturellement présents dans le sang, une coagulation intempestive avec les différents facteurs présents dans le sang, en provoquant des caillots hors de la zone hémorragique, avec des risques de thromboses.

Les inconvénients cités ci-dessus posent donc des problèmes en particulier dans le cadre d'interventions chirurgicales et/ou de situations d'urgence, où souvent la rapidité du geste opératoire est une nécessité. Il est observé que de tels inconvénients sont susceptibles d'engendrer une réunion inhomogène des tissus par formation d'amas de fibrine avant adhésion tissulaire, pouvant en outre être accentués par la rétraction du caillot, conduisant au final à une cicatrice irrégulière, propice à une déshience et à des saignements secondaires post-opératoires.

Pour remédier à ces inconvénients, la Demanderesse a cherché à mettre au point une colle biologique répondant à un double objectif conjoint. Le premier objectif est de fournir une colle biologique comprenant des facteurs plasmatiques de la coagulation, exempte de thrombine, sans risque d'une formation de fibrine avant l'application sur un tissu biologique. Le deuxième objectif répond au besoin de mettre à disposition une telle colle présentant également des capacités accrues, d'une part, d'hémostase de tissus lésés et, d'autre part, de réunion de tissus conduisant à une cicatrisation améliorée.

L'invention concerne une colle biologique liquide monocomposé, stable, à usage thérapeutique, exempte de thrombine, à base de fibrinogène, comprenant du facteur VIIa et une source d'ions calcium.

La Demanderesse a trouvé qu'il est possible de mettre à disposition un nouveau médicament (colle biologique monocomposé) : adjuvant de l'hémostase, pansement, adjuvant de cicatrisation ou obturant par formation d'emboles, pour les tissus biologiques, les plaies ou les biomatériaux, comprenant les deux facteurs plasmatiques d'intérêt ci-dessus et une source d'ions calcium.

On entend par « monocomposé », une utilisation conjointe des trois composants d'intérêt constitutifs de la colle formant ainsi un composé liquide unique, par opposition aux colles bi-composants de l'art antérieur.

Ces trois composants sont donc compatibles entres eux sous forme liquide, c'est-à-dire que leur combinaison, formant ainsi un système monocomposé, n'engendre pas la formation quasi instantanée de fibrine. Cette colle liquide monocomposé est stable car ces composants peuvent être en présence en milieu liquide en vue d'une utilisation fonctionnelle de celle-ci, et ce même au moins pendant 24 heures précédent cette utilisation, sans risque d'un déclenchement prématuré du processus de la coagulation. Cette stabilité de la colle est vérifiée, dans la mesure où on n'observe pas de fibrinoformation pendant 24 heures d'incubation à 37°C.

La colle biologique de l'invention remplit sa fonction réparatrice de tissus une fois appliquée au niveau de la plaie ou du tissu cruenté, car la thrombine est formée *in situ* par contact de la colle biologique sur la plaie ou sur le tissu cruenté

En effet, cette formation de thrombine *in situ* est assurée par le facteur VIIa qui est donc mis en contact avec tous les composants plasmatiques autorisant le déclenchement et le déroulement de la voie extrinsèque ou intrinsèque de la coagulation. Comme explicité précédemment, son activité biologique est dépendante de l'interaction avec les facteurs tissulaires de nature phospholipidique endogènes. Le complexe facteur VII activé/facteurs tissulaires en présence d'ions calcium transforme le facteur X présent dans le plasma en facteur X activé, lequel transforme la prothrombine en thrombine, enzyme responsable de la formation du caillot en générant la fibrine en présence de fibrinogène.

Il se produit alors une fibrinoformation et, par conséquent, le pouvoir adhésif de la colle biologique obtenue s'en trouve renforcé, car la fibrine se forme alors directement au niveau de la plaie ou du tissu à réparer par chirurgie, où les composants cellulaires phospholipidiques sont exposés.

Bien que le fibrinogène et les ions calcium soient déjà présents naturellement dans le sang, leur apport permet de renforcer et d'accélérer davantage encore le processus de réunion tissulaire et d'hémostase par la colle biologique de l'invention et ce même par rapport aux colles de l'art antérieur. Ceci constitue un avantage décisif de la présente colle.

La colle biologique de l'invention présente de nombreux autres avantages. Sa mise à disposition évite les inconvénients de manipulation et préparation des colles biologiques de l'art antérieur, notamment dans le cas de situations d'urgences, ce qui améliore ses aptitudes de collage tissulaire, en termes de déshience et d'absence de saignements secondaires au niveau de la cicatrice en formation. Typiquement, le processus de réunion peut être efficacement réalisé en moins d'une minute, par exemple en 30 s.

A titre d'exemple, la colle de l'invention évite la formation de « paquets » ou amas de fibrine visible au niveau d'une plaie qui s'expliquerait par une prise inhomogène de la colle, probablement à cause de la fibrine formée avant adhésion, la cicatrice formée présentant en outre un trait net, par rapport à un collage à durée égale obtenu en utilisant, par exemple, une colle biologique bi-composant à base d'une part, d'un mélange de fibrinogène et contenant du facteur XIII, et, d'autre part, d'un mélange de thrombine calcique, pour lequel on n'observe pas les effets ci-dessus.

Elle est simple à préparer et est d'une stabilité accrue dans le temps, dans la mesure où on observe par de fibrinoformation pendant 24 heures d'incubation à 37°C. A titre d'exemple, elle est donc stable sous forme liquide pendant au moins 24 heures à température ambiante, en particulier 48 heures.

Dans le cadre de l'invention, tout fibrinogène et FVIIa de l'art antérieur, de préférence plasmatique, conviennent pour réaliser la colle, à condition toutefois qu'ils soient compatibles avec les ions calcium de la colle sous forme liquide ainsi obtenue, c'est-à-dire que leur mise en contact n'engendre pas la coagulation du fibrinogène en fibrine notamment 24 heures avant utilisation. En effet, les deux ingrédients actifs, le fibrinogène et le FVIIa, doivent donc être dépourvus, outre de thrombine (FIIa) résiduelle, de facteurs de la coagulation tels qu'en présence d'ions calcium la cascade de la coagulation intrinsèque ou extrinsèque soit déclenchée. De tels facteurs de la coagulation représentent le facteur II (FII) et le FX, et, de préférence, les facteurs prothrombiniques (facteurs II, VII, IX et X).

A titre d'exemple, une teneur maximale acceptable en FII et FX dans le fibrinogène est d'environ 0,1 UI/g de fibrinogène pour chacun.

De préférence, le fibrinogène et le FVIIa de la colle ne proviennent pas d'un plasma préactivé.

Le fibrinogène, de préférence viralement sécurisé, peut être préparé par toute technique de fractionnement plasmatique connue dans l'art antérieur. Il peut s'agir du procédé décrit dans EP 0 305 243 ou encore celui mis au point par la Demanderesse dans la demande de brevet FR 05 06640 selon lequel on peut obtenir un concentré de fibrinogène. On peut également mettre en oeuvre du fibrinogène transgénique.

Le facteur XIII, de préférence viralement sécurisé, peut être isolé à partir du plasma par toute méthode développée dans l'art antérieur et il pourra avantageusement constituer la protéine accompagnante du fibrinogène lors du fractionnement du plasma. On préfère, dans ce cas, mettre en oeuvre le procédé décrit dans la demande de brevet FR 05 06640. On peut également mettre en oeuvre du facteur XIII transgénique.

La préparation du facteur VIIa, notamment sous la forme d'un concentré, de préférence viralement sécurisé, est également connue. On peut à titre d'exemple citer le brevet EP 346 241. On peut également utiliser du facteur VIIa recombinant (provenant de la Société Novo) ou transgénique.

Ces ingrédients actifs doivent toutefois répondre à certains critères de pureté, énoncés plus haut, relatifs à la présence notamment de certains autres facteurs plasmatiques.

De préférence, la colle de l'invention comprend en outre du facteur XIII. Un apport exogène de facteur XIII favorise la réticulation du réseau de fibrine et, par conséquent, son pouvoir coagulant et cicatrisant.

Le facteur XIII, en particulier sous la forme de concentré, de préférence viralement sécurisé, peut être isolé à partir du plasma par toute méthode développée dans l'art antérieur et il pourra avantageusement constituer la protéine accompagnante du fibrinogène lors du fractionnement du plasma. On préfère, dans ce cas, mettre en oeuvre le procédé décrit dans la demande de brevet FR 05 06640. On peut également mettre en oeuvre du facteur XIII transgénique.

La colle de l'invention peut être fabriquée à l'échelle industrielle grâce à la mise à disposition des deux ingrédients actifs, le fibrinogène et le FVIIa, et des ions calcium.

De préférence, la présente colle est constituée du seul mélange de fibrinogène, de FVIIa et d'ions calcium. Une telle colle exclusivement constituée de ces trois composants présente l'avantage de ne nécessiter que deux ingrédients actifs sur le plan biologique, ce qui limite notamment les coûts de préparation à l'échelle industrielle de la colle grâce à la présence d'un nombre minimal mais efficace de composants actifs.

Les composants constitutifs de la colle sont présents en des quantités efficaces permettant à la colle de répondre aux objectifs thérapeutiques recherchés.

La Demanderesse a toutefois observé que les meilleurs résultats en termes des effets recherchés mentionnés ci-dessus peuvent être obtenus lorsque les teneurs des deux ingrédients actifs et en ions calcium sont spécifiquement choisis.

Ainsi, la colle biologique comprend avantageusement du fibrinogène en une teneur comprise entre 60 et 120 mg/ml, de façon plus préférée de 80 à 100 mg/ml. Un telle teneur en fibrinogène dans la colle assure un collage au moins aussi efficace, par exemple de tissus lésés, qui se traduit par une résistance à l'arrachement du collage au moins aussi satisfaisante notamment que les colles de l'art antérieur bi-composant, telle que supérieure ou égale à 125 g/cm².

Elle comprend en particulier de 50 à 500 UI/ml, de façon plus préférée de 70 à 300 UI/ml, en particulier entre 80 et 120 UI/ml de facteur VIIa et entre 4 et 30 µmole/ml, de façon plus préférée, entre 8 et 20 µmole/ml de la source d'ions calcium.

En particulier, le facteur XIII est présent a raison de 30 UI/ml à 700 UI/ml, de préférence de 100 UI/ml à 400 UI/ml.

Les concentrations et activités sont par millilitre de solution liquide finale de colle biologique. Une telle solution peut notamment être obtenue par reconstitution des composants lyophilisés. Comme indiqué ci-dessus, ces facteurs plasmatiques présents de préférence à de telles concentrations assurent les fonctionnalités recherchées pour la présente colle biologique.

Les sources d'ions calcium représentent des composants hydrosolubles, compatibles avec un usage clinique. De préférence, ces composants sont des sels inorganiques, tels que le chlorure de calcium (CaCl₂) ou le gluconate de calcium.

Comme indiqué ci-dessus, la colle biologique de l'invention est prête à l'emploi et comprend un mélange homogène de tous les composants sous forme liquide. Elle peut également se présenter sous la forme congelée, ce qui la rend apte à un stockage prolongé sous cette forme au moins pendant 2 ans sans risque d'une fibrinoformation, qui serait observée, le cas échéant, après décongélation. II suffit d'une simple remise à température ambiante pour que la colle puisse être utilisée.

L'invention concerne également un colle biologique a usage thérapeutique, exempte de thrombine, à base de fibrinogène, comprenant du facteur VIIa et une source d'ions calcium, tels que définis précédemment, se présentant sous la forme lyophilisée, apte à un stockage prolongé. Elle peut être obtenue par la mise en oeuvre de techniques de lyophilisation connues de la colle sous forme liquide. La mise à disposition d'une telle présentation de la colle présente l'avantage décisif de ne nécessiter qu'une simple reconstitution du lyophilisat comprenant les deux ingrédients actifs et la source d'ions calcium dans un solvant ou milieu aqueux biologiquement compatible pour obtenir la colle liquide stable, cette préparation étant effectuée à l'avance en prévision de l'utilisation. De plus, une telle colle lyophilisée est très facilement stockable à température ambiante pendant au moins 2 ans sans risque d'une fibrinoformation, qui serait observée, le cas échéant, après reconstitution. Elle est aisément transportable jusqu'à un site où un patient requiert un usage de cette colle.

L'invention concerne également un kit pour la préparation de la colle biologique selon l'invention comprenant des moyens de conditionnement comprenant un lot de facteur plasmatique fibrinogène lyophilisé, un lot de facteur plasmatique FVIIa lyophilisé, un lot de source d'ions calcium sous la forme de poudre et un solvant aqueux.

Un tel kit comprenant notamment trois lots différents, individuels, sous forme sèche, chacun comprenant l'un, particulier, des composants constitutifs de la colle de l'invention, constitue en fait un produit intermédiaire dont il sera ensuite facile de réunir et de dissoudre les lots dans un solvant ou milieu aqueux biologiquement compatible, tel que l'eau purifiée pour injection (PPI), pour l'obtention de la colle biologique liquide stable de l'invention, concentrée au besoin, qui peut ensuite être congelée. L'avantage de mettre à disposition un tel kit est la possibilité d'un stockage prolongé des trois lots de composants différents au moins pendant 2 ans à température ambiante, sans l'observation d'une fibrinoformation après reconstitution, et l'obtention de la colle biologique liquide et stable par simple dissolution.

Les moyens de conditionnement constituent en fait des moyens permettant de stocker en particulier les composants de la colle, de préparer et de distribuer la colle avant utilisation. Ils comprennent en outre avantageusement au moins un récipient pour au moins trois des composants différents de la colle, dont, de préférence, au moins un pour chaque composant. Chaque récipient est destiné à recevoir l'un des composants qui est ensuite dissous dans le solvant aqueux. Les récipients peuvent être des flacons en divers matériaux de type verre et polymères biologiquement compatibles.

De préférence, les moyens de conditionnement peuvent être avantageusement un récipient unique contenant au moins les trois composants. Un tel conditionnement présente l'avantage qu'une simple dissolution de l'ensemble fournit directement la colle liquide prête à l'emploi.

Avantageusement, le kit comprend également un dispositif de distribution de la colle liquide une fois préparée par dissolution des lots ci-dessus avec le solvant aqueux. Un tel dispositif représente par exemple une seringue de volume approprié en fonction de la dose de colle à délivrer comportant une aiguille fine, typiquement de diamètre inférieur à 2 mm, en particulier inférieur à 1 mm, ou bien un cathéter classique.

De préférence, le kit ci-dessus comporte un lot bi-composant de mélange des lots desdits facteurs plasmatiques lyophilisés, et le lot de source d'ions calcium sous la forme de poudre. Ce kit comprend donc, d'une part, un lot de mélange lyophilisé des facteurs plasmatiques fibrinogène et FVIIa de l'invention, et, d'autre part, un lot de la source d'ions calcium sous forme de poudre, susceptible d'être également sous la forme d'un lyophilisat, qu'il suffira simplement de réunir et de mélanger avec un milieu aqueux, tel que l'eau PPI, avant utilisation. En variante, le kit peut comprendre un lot de mélange lyophilisé facteurs plasmatiques, et, d'autre part, un lot de la source d'ions calcium sous forme de solution aqueuse.

De préférence, le kit de l'invention est caracterisé par le fait que chaque lot de facteur plasmatique fibrinogène ou FVIIa lyophilisé, ou le lot bi-composant du mélange desdits lots des facteurs plasmatiques lyophilisés comprend des constituants d'une formulation stabilisante de lyophilisation pharmaceutiquement acceptable. Il en est de même de la colle biologique lyophilisée.

En effet, les différents lyophilisats des facteurs plasmatiques sont obtenus par lyophilisation des concentrés ou solutions liquides des facteurs plasmatiques ou de chacun des ces facteurs, selon des techniques classiquement mises en oeuvre, comprenant avantageusement, à cet effet, une formulation stabilisante de lyophilisation, telle que décrite dans la demande de brevet FR 04 02001 déposée par la Demanderesse. Dans ce cas, la formulation stabilisante représente avantageusement un mélange d'arginine, d'au moins un acide amine hydrophobe et de citrate trisodique et peut en outre être additionnée de glycine et/ou de lysine. Avantageusement, les concentrations en chacun des additifs, par litre de concentré de protéines, sont les suivantes:
- arginine, de 25 à 50 g/l et de préférence de 35 à 45 g/l (en référence au brevet US 5 399 670);
- citrate trisodique, de 0,5 à environ 12 g/l;
- leucine, isoleucine ou leur mélange, de 5 à 15 g/l et de préférence de 9 à 11 g/l; et
- glycine et/ou lysine, chacune de 1 à 5 g/l et de préférence chacune de 1,5 à 2,5 g/l.

La formulation stabilisante peut également, en tant que de besoin, comprendre des adjuvants stabilisants connus dans la technique.

Par conséquent, l'invention concerne également l'utilisation du kit selon l'invention pour la préparation d'une colle biologique liquide, puis éventuellement congelée, par reconstitution des trois lots dudit kit dans un solvant aqueux biologiquement compatible suivie, le cas échéant, d'une congélation.

L'invention concerne également une colle biologique telle que décrite plus haut pour utilisation comme médicament. Dans le cas où la colle est stockée sous la forme congelée, il suffira, préalablement à l'utilisation, de dégeler celle-ci. Dans le cas d'une colle lyophilisée, la reconstitution dans un solvant aqueux biologiquement compatible permet de l'utiliser pour les effets recherchés.

La colle biologique de l'invention est donc utilisée pour la préparation d'un médicament destiné à l'hémostase et/ou la cicatrisation de tissus biologiques lésés, tels que la peau ou tout organe susceptible d'interventions chirurgicales (rate, foie, poumons intestins etc.), qui, comme explicité précédemment, peuvent représenter les tissus cruentés ou les plaies hémorragiques, dont le saignement peut même être très faible dans la mesure où tous les facteurs nécessaires à la cascade de la coagulation sont présents. La colle peut être utilisée, en présence de plasma, pour la préparation d'un médicament destiné à traiter les tissus lésés, par exemple réunir ces tissus, choisis dans le groupe constitué par le cartilage, le collagène, l'os et la poudre d'os.

De plus, en présence de plasma, elle est également utilisée pour la préparation d'un médicament destiné à réunir les biomatériaux choisis dans le groupe constitué par les alginates ou l'acide polylactique. La présence de plasma est par conséquent nécessaire dans certains cas pour l'apport exogène des facteurs plasmatiques afin d'initier la cascade de la coagulation, et, de préférence, est du plasma compatible ou autologue. Cet apport pourra notamment être effectué dans le cadre d'interventions chirurgicales classiques, en stomatologie ou odontologie.

Le tissu biologique peut également provenir de cellules-souches mises en culture et différenciées.

Bien que la colle de l'invention soit un médicament, en particulier un pansement à application locale, c'est-à-dire à usage externe sur une plaie ou autre comme décrit plus haut, il n'est pas exclu que le médicament puisse également être une gélule adaptée, gastrorésistante ou non, dans laquelle la colle biologique est sous forme sèche, et ingérée pour le traitement d'un saignement digestif.

La stabilité d'au moins 24 heures de la colle de l'invention lui permet de rester fluide et donc de pouvoir être utilisée pour la préparation d'un médicament destiné à emboliser les vaisseaux sanguins nourriciers d'une cible tumorale. Ceci peut être avantageusement effectué par voie d'injection utilisant un cathéter classique jusqu'à ces cibles tumorales pour ainsi « assécher » la tumeur.

La colle permet d'apporter une hémostase à travers un système endoscopique utilisé en microchirurgie (prélèvements, biopsies, excision de polypes etc.).

La fluidité de la présente colle, liée notamment à sa stabilité, rend possible son utilisation à travers d'une aiguille fine, typiquement de diamètre inférieur à 1 mm, en particulier inférieur à 0,5 mm, pour une application en chirurgie sous microscope, par exemple ophtalmique.

L'exemple suivant illustre l'invention sans toutefois en limiter la portée.

### Exemple

On prépare 2 ml d'un échantillon de colle biologique de l'invention (échantillon A) comprenant du fibrinogène à une concentration de 80 mg/ml, 100 UI/ml de facteur VIIa et 5 µmole/ml de chlorure de calcium qui sont introduits dans une seringue unique.

On prépare également un échantillon B standard de colle biologique de l'art antérieur comprenant, d'une part, un mélange B1 constitué de fibrinogène à une concentration de 80 mg/ml et contenant 100 UI/ml de facteur XIII, et, d'autre part, un mélange B2 constitué de thrombine calcique à 500 UI/ml. Les mélanges B1 et B2 respectif sont introduits dans deux seringues distinctes dont les extrémités sont agencées pour comporter une aiguille unique permettant ainsi la réunion des mélanges B1 et B2.

Un lapin anesthésié est soumis à une laparotomie. Le foie est exposé et on procède à une première incision de l'organe afin de provoquer un saignement sur cette tranche. Après tamponnage de l'excès de sang par une compresse, on applique immédiatement l'échantillon B de colle biologique sur la tranche incisée par expulsion simultanée du contenu des deux seringues par l'aiguille unique qui permet la préparation d'un mélange homogène de B1 et de B2. Les berges de la plaie sont maintenues réunies pendant 30 secondes pour la prise de la colle biologique.

Après cicatrisation, il est observé que la cicatrice obtenue présente des irrégularités et des « paquets » de fibrine visible. Ces amas sont dus à une prise inhomogène, probablement à cause de la fibrine formée avant adhésion.

Une seconde incision est effectuée à un autre endroit du foie qui est chirurgicalement traitée de la même façon que précédemment. L'échantillon A de colle biologique selon l'invention, contenue dans une seule seringue, est appliqué sur la tranche incisée et les berges de la plaie sont maintenues réunies pendant 35 secondes pour la prise de la colle biologique.

Après cicatrisation, il est observé que la cicatrice obtenue présente un trait net et résiste bien à la tentative de déshience. Aucune trace de fibrine excédentaire n'est visible, ce qui montre que l'excès de colle n'a pas coagulé en l'absence de plaie opératoire et donc sans présence de facteurs tissulaires.

## Revendications

1. Colle biologique liquide monocomposé, stable, à usage thérapeutique, exempte de thrombine, à base de fibrinogène, comprenant du facteur VIIa.

2. Colle biologique selon la revendication 1, constituée du seul mélange de fibrinogène et de FVIIa.

3. Colle biologique selon la revendication 1 ou 2, comprenant une teneur en fibrinogène comprise entre 60 et 120 mg/ml, avantageusement comprise entre 80 et 100 mg/ml, de solution liquide finale de colle biologique.

4. Colle biologique selon l'une des revendications 1 à 3, comprenant de 50 à 500 UI de facteur VII activé par millilitre de solution liquide finale de colle biologique (50-500 UI/ml), avantageusement de 70 à 300 UI/ml de facteur VII activé, en particulier de 80 à 120 UI/ml.

5. Colle biologique selon l'une des revendications 1 et 3 à 4, comprenant en outre du facteur XIII.

6. Colle biologique selon la revendication 5, dans laquelle le facteur XIII est présent à raison de 100 UI à 400 UI par millilitre de solution liquide finale de colle biologique (100 UI/ml-400 UI/ml).

7. Colle biologique selon l'une des revendications 1 à 6 se présentant sous la forme congelée, apte à un stockage prolongé.

8. Colle biologique à usage thérapeutique, exempte de thrombine, à base de fibrinogène, comprenant du facteur VIIa, tels que définis selon l'une des revendications 1 à 7, se présentant sous la forme lyophilisée, apte à un stockage prolongé.

9. Colle biologique selon la revendication 8, comprenant en outre un mélange d'arginine, d'au moins un acide aminé hydrophobe et de citrate trisodique.

10. Kit pour la préparation de la colle biologique selon l'une des revendications 1 à 7 comprenant des moyens de conditionnement comprenant un lot de facteur plasmatique fibrinogène lyophilisé, un lot de facteur plasmatique FVIIa lyophilisé et un solvant aqueux.

11. Kit selon la revendication 10, comportant un lot bi-composant de mélange desdits lots des facteurs plasmatiques lyophilisés.

12. Kit selon l'une des revendications 10 ou 11, dans lequel chaque lot de facteur plasmatique fibrinogène ou FVIIa lyophilisé, ou le lot bi-composant du mélange desdits lots des facteurs plasmatiques lyophilisés comprend en outre un mélange d'arginine, d'au moins un acide aminé hydrophobe et de citrate trisodique.

13. Utilisation d'un kit selon l'une des revendications 10 à 12 pour la préparation d'une colle biologique telle que définie selon l'une des revendications 1 à 9 par reconstitution des lots dudit kit dans un solvant aqueux biologiquement compatible suivie, le cas échéant, d'une congélation.

14. Colle biologique telle que définie selon l'une des revendications 1 à 9 pour utilisation comme médicament.

15. Utilisation de la colle biologique selon l'une des revendications 1 à 9, pour la préparation d'un médicament destiné à l'hémostase et/ou la cicatrisation de tissus biologiques lésés tels que les tissus cruentés ou les plaies hémorragiques.

16. Utilisation selon la revendication 15, dans laquelle les tissus lésés sont la peau ou tout organe susceptible d'interventions chirurgicales.

17. Utilisation selon la revendication 15 ou 16, dans laquelle ledit médicament est un pansement à application locale ou une gélule dans laquelle ladite colle biologique est sous la forme sèche.

18. Utilisation de la colle biologique selon l'une des revendications 1 à 9 et de plasma, pour la préparation d'un médicament destiné à _{:}
i) traiter les tissus lésés choisis dans le groupe constitué par le cartilage, le collagène, l'os et la poudre d'os ;
ii) réunir les biomatériaux choisis dans le groupe constitué par les alginates ou l'acide polylactique ; et/ou
iii) emboliser les vaisseaux sanguins nourriciers d'une cible tumorale.
